# EUROPÄISCHE PATENTSCHRIFT

(11) **EP 1 913 381 B1**
(45) Veröffentlichungstag und Bekanntmachung des Hinweises auf die Patenterteilung: **09.09.2015**
(21) Anmeldenummer: 06761233.3
(22) Anmeldetag: 21.07.2006
(51) Int. Cl.: G01N 33/36

(54) **GARNPRÜFGERÄT**
THREAD TESTING APPLIANCE
APPAREIL DE CONTROLE DE FIL

(30) Priorität: 30.07.2005 CH 12732005
(43) Veröffentlichungstag der Anmeldung: 23.04.2008
(73) Patentinhaber: Uster Technologies AG, 8610 Uster (CH)
(72) Erfinder: SCHULTHESS, Jürg, CH-8610 Uster (CH)
(86) Internationale Anmeldenummer: PCT/CH2006/000382
(87) Internationale Veröffentlichungsnummer: WO 2007/014475

(56) Entgegenhaltungen:
- EP-A- 0 467 159
- WO-A-03/031699
- CH-A5- 563 021
- DE-A1- 2 340 417
- DE-A1- 3 028 453
- DE-A1- 4 422 596
- US-A- 4 990 793

## Beschreibung

Die Erfindung betrifft eine Vorrichtung zur automatischen Bestimmung von Kenngrössen an textilem Prüfgut wie Garnen, Vorgarnen und Bändern, gemäss dem Oberbegriff des ersten Patentanspruchs. Ferner betrifft die Erfindung ein Verfahren zur Entfernung von unerwünschten Partikeln wie losen Fasern, Schmutz oder Staub aus einer Vorrichtung zur automatischen Bestimmung von Kenngrössen an textilem Prüfgut wie Garnen, Vorgarnen und Bändern, gemäss dem Oberbegriff des weiteren unabhängigen Patentanspruchs.

### STAND DER TECHNIK

Aus der CH-671'105 ist eine solche Vorrichtung bekannt, mit der beispielsweise Massenschwankungen von Garnen, Vorgarnen oder Bändern laufend gemessen werden können, währenddem eine Vorschubeinrichtung das Garn, Vorgarn oder Band in seiner Längsrichtung durch ein Messorgan hindurch bewegt. Vor und/oder nach dem Messorgan sind in bekannter Weise Führungsmittel vorgesehen, die das Prüfgut so führen, dass es während seiner Bewegung in Längsrichtung durch das Messorgan möglichst keine seitlichen Auslenkungen ausführt, oder mit anderen Worten möglichst nicht schwingt.

Ein Nachteil solcher Vorrichtungen besteht darin, dass sich an bestimmten Stellen des ortsfest angeordneten Messorgans wie auch der ortsfesten Führungsmittel unerwünschte Partikel wie lose Fasern, Staub und Schmutz ablagern. Solche Partikel werden vom Prüfgut mitgerissen oder vom Prüfgut abgegeben. Dies trifft umso mehr zu, je höher die Geschwindigkeit ist, mit der das Prüfgut bewegt wird, und je enger ein Messkanal oder ein Messspalt im Messorgan ist. Da der Staub, der Schmutz und die losen Fasern sich genau dort ablagern, wo durch das laufende Prüfgut keine Berührung stattfindet, findet man diese Ablagerungen üblicherweise an bestimmten ausgezeichneten Stellen neben der Bahn des Prüfguts. Solche Stellen erfüllen gewisse Bedingungen, so dass die Ablagerungen ungestört aufgebaut werden können. Die Ablagerungen beeinträchtigen die Funktion des Messorgans, insbesondere wenn es sich um ein optisches Messorgan handelt. Sie können aber auch die Bewegung von mechanischen Teilen, z. B. Umlenk- oder Führungsrollen, behindern oder den Pfad des Prüfguts verstopfen.

Mit der unerwünschten Ablagerung von Fremdstoffen auf dem Messkondensator selbst befasst sich die CH-563'021 A5. Sie schlägt vor, eine Luftströmung über die Oberseite des Messkondensators zu führen, um die Ablagerung zu verhindern. Die Luftströmung wird erzeugt, indem im Innern des Apparategehäuses mittels eines Ventilators einen Überdruck zu erzeugen. Oberhalb des Messkondensators weist das Apparategehäuse einen Schlitz auf, durch den Luft ausströmt und Faserflug vom Messkondensator wegbläst. Alternativ kann die Luft durch einen Führungskanal aus dem Apparategehäuse geleitet und entlang der Frontseite des Gerätes ausgeblasen werden. Das Ausblasen von Luft aus dem Apparategehäuse hat den Nachteil, dass die weggeblasenen Fremdstoffe unkontrolliert in die Umgebung gelangen und sich möglicherweise an anderen Orten ablagern, wo sie ebenfalls stören könnten. Ausserdem ist die aus dem Apparategehäuse wegen der im Gehäuse von elektrischen Bauteilen abgegebenen Wärme wärmer als die Umgebungsluft. Diese zusätzliche Wärme kann das üblicherweise sehr empfindliche Messorgan beeinflussen und zu Fehlmessungen führen.

Die DE-30'28'453 A1 offenbart eine Spinnmaschine, in welcher an jeder Spinnstelle ein Garn aus zwei Faserbändern gesponnen wird. Jeder Spinnstelle ist ein Fühler zugeordnet, der auf den Bruch eines der beiden Faserbänder anspricht und im Gefolge jedes solchen Ansprechens die Unterbrechung des Spinnens auslöst. Der Fühler befindet sich in einer Absaugvorrichtung, die alle nicht in das Garn eingedrehten Fasern einsaugt. In einem Absaugrohrstutzen der Absaugvorrichtung ist eine Lichtschranke angeordnet, welche die Dichte der abgesaugten Fasern misst. Überschreitet der Messwert einen vorgegebenen Schwellenwert, so liegt ein Faserbandbruch vor, und die beiden Faserbänder werden von einer Trennvorrichtung durchgeschnitten.

Die EP-0'467'159 A1 stellt sich die Aufgabe, eine Spinnmaschine zu reinigen. Zu diesem Zweck sind im Bereich des Streckwerkes und der Luftwirbeldüse Absaugrohre angeordnet, welche nicht versponnenen Faserflug absaugen.

### DARSTELLUNG DER ERFINDUNG

Durch die Erfindung soll nun eine Vorrichtung der genannten Art vorgeschlagen werden, die die genannten Nachteile nicht aufweist und die die Ablagerung loser Fasern sowie von Staubund Schmutzteilchen verringert. Ferner soll ein entsprechendes Verfahren zur Entfernung von unerwünschten Partikeln wie losen Fasern, Schmutz oder Staub aus einer Vorrichtung zur automatischen Bestimmung von Kenngrössen an textilem Prüfgut angegeben werden.

Diese und andere Aufgaben werden durch die Vorrichtung und das Verfahren gelöst, wie sie in den unabhängigen Patentansprüchen definiert sind. Vorteilhafte Ausführungsformen sind in den abhängigen Patentansprüchen angegeben.

Die Erfindung sieht an mindestens einer Stelle längs eines Pfades oder einer Bahn, die das Prüfgut durchläuft, Mittel zur Entfernung von unerwünschten Partikeln, die aus dem Prüfgut selbst stammen oder die vom Prüfgut mitgerissen werden, vor. Die Mittel zur Entfernung von unerwünschten Partikeln beinhalten mehrere entlang des Pfades angeordnete und voneinander beabstandete Absaugorgane, die geeignet sind, einen Unterdruck zu erzeugen, mittels welchen die unerwünschten Partikel aus dem Pfad absaugbar sind.

Vorzugsweise soll mindestens eine Untermenge der Absaugorgane derart ausgebildet und angeordnet sein, dass im Pfad eine Luftströmung aufbaubar ist, die das Prüfgut erfasst. Der Messkanal und die Absaugorgane sollen so aufeinander abgestimmt sein, dass die Luftströmung in mindestens einem Abschnitt des Messkanals die Kanalwand des Messkanals überströmt. Die Absaugorgane können, in Bewegungsrichtung des Prüfgut gesehen, an mehreren Stellen vor der Antriebsvorrichtung angeordnet sein. Solche Vorrichtungen zur Entfernung sind dann besonders wirksam, wenn sie im Bereich eines Messorgans oder im Bereiche der Zuführung des Prüfguts angeordnet sind. Alternativ dazu kann es aber, je nach Art des Messorgans, vorteilhaft sein, die Absaugorgane ausserhalb des Bereichs eines Messorgans anzuordnen.

In einer bevorzugten Ausführungsform ist entlang des Pfades ein im Wesentlichen U-formiger Messkanal vorgesehen, in dem mindestens ein Messorgan und die Mittel zur Entfernung von unerwünschten Partikeln angeordnet sind. Die letzteren beinhalten einen Luftkanal, der sich längs des Pfades erstreckt und in dem als Absaugorgane ausgebildete Öffnungen angeordnet sind. Alternativ kann mindestens ein Absaugorgan als mindestens ein Rohrstück mit einer Öffnung ausgebildet sein, die auf eine Zone im Bereich des Pfades ausgerichtet ist. Ein Absaugorgan kann auch als Ventilator ausgebildet sein, der einen zu ihm gerichteten Luftstrom um das Prüfgut und das Messorgan erzeugt.

Die durch die Erfindung erreichten Vorteile sind insbesondere darin zu sehen, dass die Vorschubeinrichtung das Prüfgut mit wesentlich höherer Geschwindigkeit durch das Messorgan und die Führungen bewegen kann, ohne dass sich nennenswerte Schmutzablagerungen bilden. Eine solche Geschwindigkeit kann beispielsweise 800 m/min betragen und die bisher üblichen Werte von 400 m/min wesentlich übertreffen. Dadurch ist es nun auch möglich, die Leistung der vorhandenen Elektronik, die die Signale aus dem Messorgan weiterverarbeitet, z. B. indem sie periodisch anfallende Messwerte mit Grenzwerten vergleicht und klassiert, besser auszunützen und die Messzeiten zu verkürzen. Ein weiterer Vorteil ist darin zu sehen, dass auf diese Weise die erfindungsgemässe Vorrichtung auch dazu beiträgt, den Arbeitplatz in einem textiltechnischen Labor sauber zu halten.

### AUFZÄHLUNG DER ZEICHNUNGEN

Im Folgenden wird die Erfindung anhand eines Ausführungsbeispiels und mit Bezug auf die beigefügten Zeichnungen veranschaulicht. Es zeigen:
Fig. 1 ein Gamprüfgerät in perspektivischer Darstellung,
Fig. 2 einen Messkanal in perspektivischer Darstellung,
Fig. 3 einen Querschnitt durch den Messkanal gemäss Fig. 2,
Fig. 4 eine Zuführung in perspektivischer Darstellung,
Fig. 5 einen Eingang zum Messkanal in perspektivischer Darstellung und
Fig. 6 eine Aufsicht auf den Eingang zum Messkanal.

### AUSFÜHRUNG DER ERFINDUNG

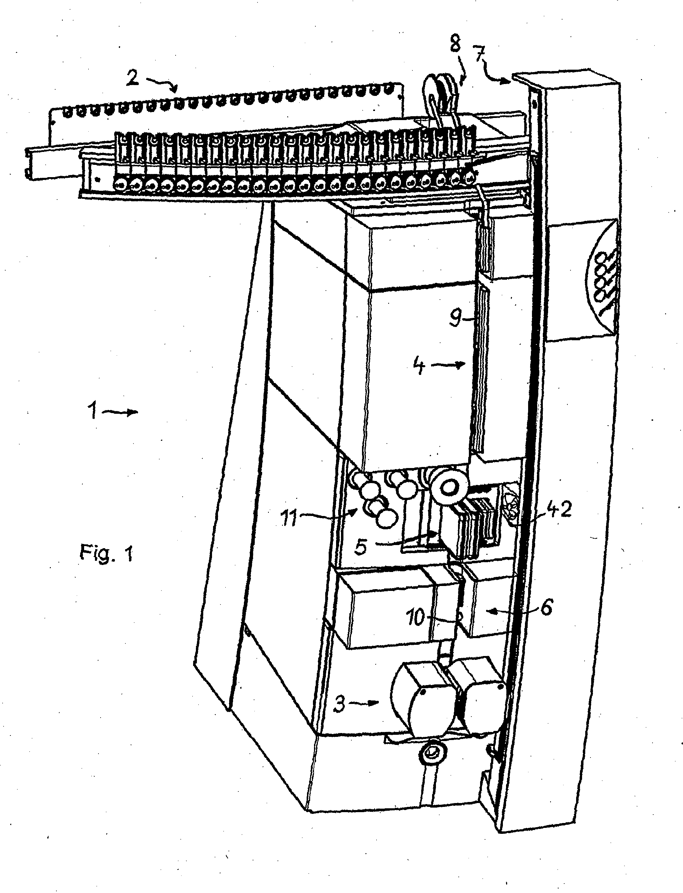

**Figur 1** zeigt eine erfindungsgemässe Vorrichtung 1 zur automatischen Bestimmung von Kenngrössen an textilem Prüfgut wie Garnen, Vorgarnen und Bändern, welche man kurz auch als Garnprüfgerät bezeichnet. Diese Vorrichtung 1 weist in an sich bekannter Weise eine Zuführung 2, eine Vorschubeinrichtung 3, hier mehrere Messorgane 4, 5, 6 und eine Einlegevorrichtung 7 zum automatischen Einlegen des Prüfguts in die Messorgane 4, 5, 6 auf. Ein Prüfgut ist hier nicht gezeigt, wohl aber erkennt man einen Pfad 8 für das Prüfgut, der durch die hintereinander angeordneten Elemente wie Zuführung 2, Messorgane 4, 5, 6 und Vorschubeinrichtung 3 gebildet wird. Im Bereiche der Messorgane 4 und 6 erkennt man auch einen im Wesentlichen U-förmigen Messkanal 9 und 10. Im Bereiche des Messorgans 5, das hier als Messkondensator für eine kapazitive Messung z. B. der Masse des Prüfguts ausgebildet ist, erkennt man ferner eine Gruppe 11 aus Umlenkrollen, die dazu geeignet sind, beispielsweise ein Faserband seitlich in den Pfad 8 einzuführen, um das Faserband im Messorgan 5 und/oder 6 zu prüfen.

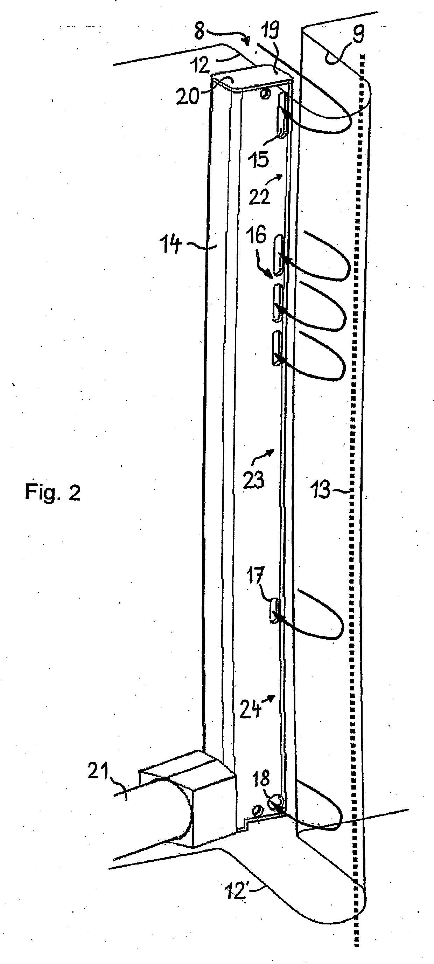

**Figur 2** zeigt den Pfad 8 im Bereich des Messkanal 9, wie er aus dem Inneren der Vorrichtung 1 gesehen (was praktisch nicht möglich ist) erscheint. Deshalb ist der Messkanal 9 hier durchsichtig dargestellt. Man erkennt seinen U-förmigen Querschnitt an seinen oberen und unteren Begrenzungen 12, 12'. Eine unterbrochene Linie markiert ein Prüfgut 13, hier beispielsweise ein Garn. Seitlich in den Messkanal 9 mit der Begrenzung 12 ist ein Luftkanal 14 so eingelassen, dass er mit einem Teil 19 in den Messkanal 9 hinausragt und mit einem Teil 20 in das Innere der Vorrichtung 1 hineinragt. Der Luftkanal 14 ist unten beispielsweise an eine Leitung 21 angeschlossen, die wiederum mit einer hier nicht gezeigten, an sich bekannten Saugpumpe verbunden ist. Öffnungen 15, 16, 17 und 18 sind in Abständen 22, 23 und 24 zueinander angeordnet. Die Abstände 22, 23, 24 können an die Lage der einzelnen (in Fig. 2 nicht dargestellten) Messorgane angepasst sein. Je nach Art des Messorgans kann es vorteilhaft sein, die Öffnungen 16, 17 im Bereich eines Messorgans anzubringen. Andererseits gibt es Messorgane, deren Funktion durch einen Luftstrom beeinträchtigt werden kann; in diesem Fall bringt man die Öffnungen 15, 18 vorzugsweise ausserhalb des Bereichs des Messorgans an. Der Luftkanal 14 mit seinen Öffnungen 15, 16, 17 und 18 bildet hier Mittel zur Entfernung von unerwünschten Partikeln, wobei diese Partikel abgesaugt werden.

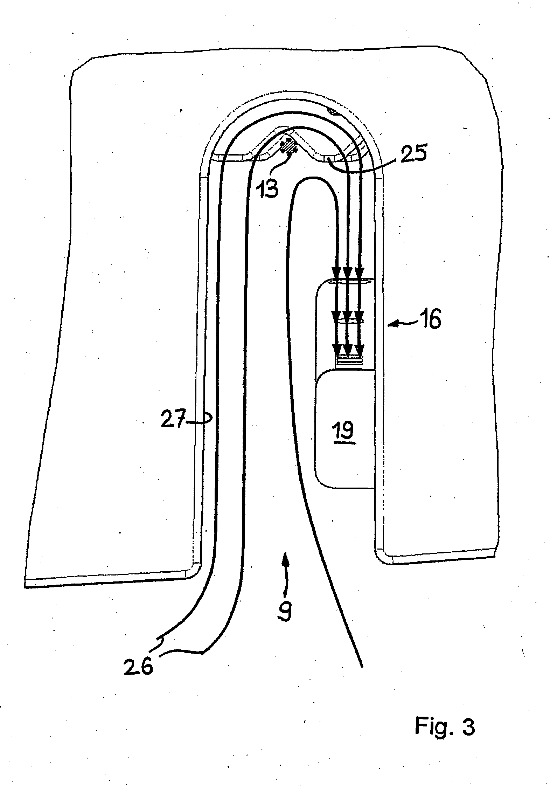

**Figur 3** zeigt einen Querschnitt durch den Messkanal 9 oberhalb der Öffnungen 16. Man erkennt hier etwa auf der Höhe der Öffnungen 16 ein Messorgan 25, das in den Messkanal 9 hinausragt, sowie das Prüfgut 13 im Schnitt. Ebenfalls sichtbar ist der in den Messkanal 9 ragende Teil 19 des Luftkanals 14. Mit Pfeilen 26 ist die Luftströmung im Messkanal 9 veranschaulicht, die die Kanalwand 27 überströmt, das Prüfgut 13 und das Messorgan 25 erfasst und dann in die Öffnungen 16 eintritt.

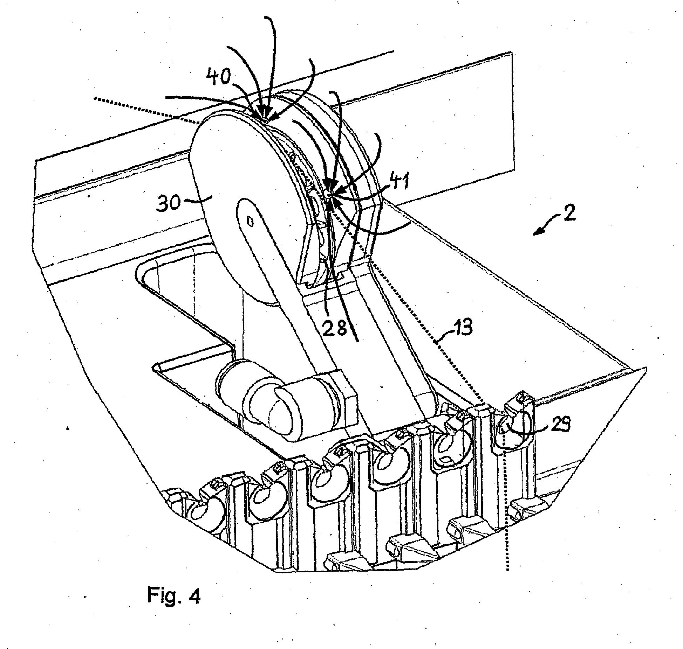

**Figur 4** zeigt einen Teil der Zuführung 2 für das Prüfgut 13 mit einem teilweise erkennbaren Umlenkrad 28, das zugeführtes Prüfgut 13 in eine Öse 29 leitet, die auf den Messkanal 9 ausgerichtet ist, das Prüfgut umlenkt und somit mit dem Messkanal 9 einen Teil des Pfades 8 für das Prüfgut bildet. Das Umlenkrad 28 kann sich frei drehen und ist in einem Ausleger 30 gelagert. Als Mittel zur Entfernung unerwünschter Partikel sind hier Öffnungen 40 und 41 vorgesehen, die an eine (in Fig. 4 nicht dargestellte) Unterdruckquelle angeschlossen sind.

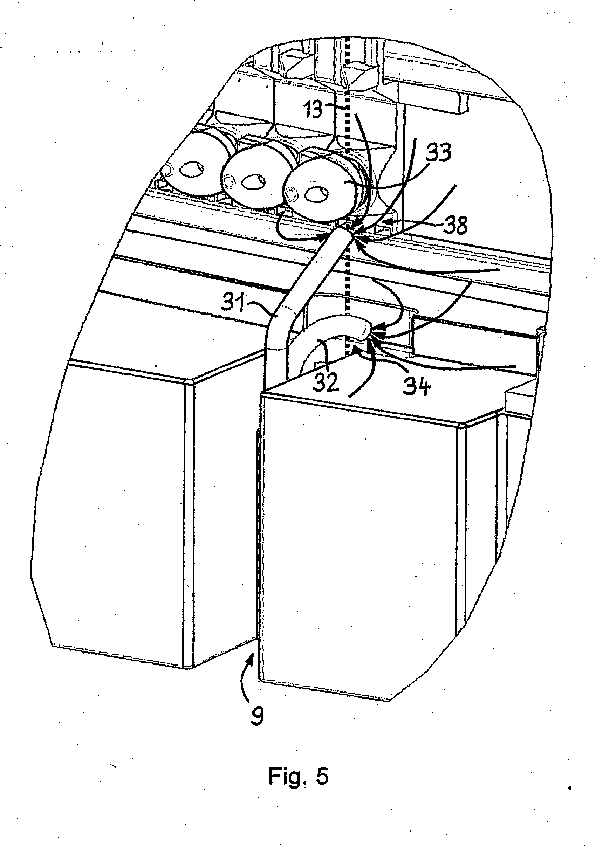

**Figur 5** zeigt einen weiteren Abschnitt des Pfades 8 vor dem Eintritt des Prüfguts 13 in den Messkanal 9. Als Mittel zur Entfernung unerwünschter Partikel sind hier zwei Rohrstücke 31, 32 vorgesehen, deren Öffnungen die Umgebung einer Klemme 33 für das Prüfgut 13, sowie den Eingang 34 zum Messkanal 9 beaufschlagen. Die Klemme 33 ist ein Teil der Zuführung 2. Sie ist während der Messungen am Prüfgut 13 geöffnet und klemmt somit das Prüfgut 13 nicht. Die Klemme 33 kommt beim Wechsel von einem Prüfgut zu einem anderen Prüfgut zum Einsatz.

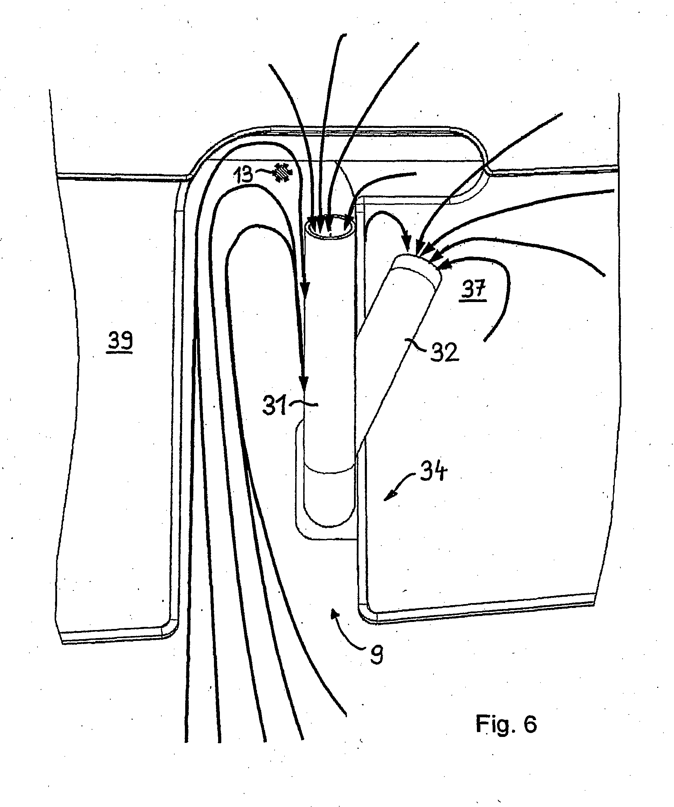

**Figur 6** zeigt den Eingang'34 zum Messkanal 9 in Aufsicht mit dem Prüfgut 13 im Querschnitt. Man erkennt hier, dass das zweite Rohrstück 32 mit seinem Ende so ausgerichtet ist, dass es den Eingang 34 mit seiner waagrechten Fläche 37 beaufschlagt. Das erste Rohrstück 31 dagegen ist auf einen Bereich 38 (siehe Fig. 5) am Ausgang der Klemme 33 ausgerichtet. Die Rohrstücke 31, 32 können beispielsweise am Luftkanal 14 befestigt sein und auch über den Luftkanal 14 an eine Unterdruckquelle angeschlossen sein.

Nachfolgend soll die Wirkungsweise der erfindungsgemässen Vorrichtung 1 erläutert werden. Wenn das Prüfgut 13 durch die Einlegevorrichtung 7 in an sich bekannter Weise in die erfindungsgemässe Vorrichtung 1 eingelegt ist, erstreckt es sich über das Umlenkrad 28, die Messkanale 9 und 10, das Messorgan 5 und die Vorschubeinrichtung 3. Dann können die Messungen am Prüfgut 13 beginnen, und die vorhandenen Messorgane 4, 5, 6 können beispielsweise Parameter wie Masse, Durchmesser, Haarigkeit, Gehalt an Fremdstoffen usw. am laufenden Prüfgut 13 messen. Das Prüfgut 13 wird durch die Vorschubeinrichtung 3 dem Pfad 8 entlang gezogen, wobei es über die Umlenkrolle 28, durch die Öse 29 und durch die verschiedenen Messorgane 4, 5, 6 in seiner Längsrichtung hindurchbewegt wird. Im Ausleger 30 und im Umlenkrad 28 können sich bereits Ablagerungen von Staub, Schmutz oder losen Fasern festsetzen, die teilweise auch durch das Umlenkrad 28 bewirkt sind. Die Öffnungen 40, 41 saugen beispielsweise solche Ablagerungen laufend ab, was bewirkt, dass von dem Ausleger 30 mit dem Umlenkrad 28 auch keine Ablagerungen durch das Prüfgut 13 selbst weiterbefördert werden. Beim nachfolgenden Durchgang des Prüfguts 13 durch die ortsfeste Öse 29 reibt sich das Prüfgut 13 daran und es besteht die Möglichkeit, dass dort vom Prüfgut 13 Schmutz oder Faserteile losgelöst werden, die sich dann weiter unten, am Eingang 34 zum Messkanal 9, ablagern. Dort werden nun solche Ablagerungen von den waagrechten Flächen 37, 39 und aus der Umgebung der Klemme 33 von der Luftströmung erfasst, die in die Rohrstücke 31, 32 eintritt. Im nachfolgenden Messkanal 9 bewirken Öffnungen 15-18 eine Reinigung des Messkanals 9. Abstände 22, 23, 24 zwischen den Öffnungen 15-18 sind den entlang dieses Messkanals 9 angeordneten Messorganen 4 angepasst. Dabei kann man davon ausgehen, dass jedes dieser Messorgane 4 einen bestimmten Parameter des Prüfguts 13 aufnimmt oder misst. Solche Messorgane 4 können Verengungen im Querschnitt des Messkanals 9 bilden, wie dies beispielsweise in Fig. 3 mit dem Messorgan 25 angedeutet ist, das in den Messkanal 9 hinausragt. Da das Prüfgut 13 aufgrund seiner Bewegung in seiner Längsrichtung entlang dem Pfad 8 und dem Messkanal 9 schwingen kann, können sich auch Querbewegungen des Prüfguts 13 ergeben. Im Bereiche solcher Verengungen besteht somit eine erhöhte Gefahr, dass Ablagerungen gebildet werden, wenn das Prüfgut 13 z. B. das Messorgan 25 berührt. Deshalb sind auch die Öffnungen 16, 17 vorzugsweise gerade in solchen Bereichen angeordnet, um zu vermeiden, dass beispielsweise lose Fasern vom Prüfgut 13 mitgerissen werden und sich im Pfad 8 weiter unten ablagern. Da dies trotzdem noch möglich ist, sind weiter unten eben weitere Öffnungen 18 vorgesehen.

Wie man aus den Figuren 2 und 3 erkennen kann, sollen die Ablagerungen in einer geordneten Luftströmung 26 beispielsweise entlang der Kanalwand 27 in die Öffnungen 15-18 geführt werden. Dazu sollen der Luftkanal 14 mit dem Messkanal 9 und den Öffnungen 15-18 so aufeinander abgestimmt sein, dass die Luftströmung 26 in mindestens einem Kanalabschnitt die gesamte Kanalwand 27 des Messkanals 9 überströmt und in die Öffnungen 15-18 mündet. In jedem Falle soll die erfindungsgemässe Vorrichtung lockere Partikel laufend entfernen, damit sie sich später an unerwünschten Stellen längs des Pfades nicht mehr absetzen. Insbesondere im Bereiche von Garnführungen, die wiederum in unmittelbarer Nachbarschaft zu den Messorganen angeordnet sind, bildet sich durch Reibung mit dem laufenden Garn 13 Staub, der laufend entfernt werden soll.

Wie aus der Fig. 1 erkennbar ist, kann ein Absaugorgan zum Absaugen von unerwünschten Partikeln auch als Ventilator 42 ausgebildet sein. Der Ventilator 42 ist an geeigneter Stelle, hier neben dem Messorgan 5, angebracht und saugt Luft aus der Umgebung an. Dadurch werden die unerwünschten Partikel entfernt.

Der Messkanal 9, 10 kann ausser der besonders günstigen U-Form auch andere Formen haben, sofern sich diese zur Aufnahme von Messorganen eigenen und eine solche Luftströmung begünstigen, die die unerwünschten Partikel möglichst wirksam entfernen lässt. Es ist ferner vorteilhaft, die Zahl der Öffnungen für die Luft längs des Pfades in Bewegungsrichtung des Garnes nach und nach zu verringern. Somit soll die Dichte der Öffnungen in einem ersten Teil des Pfades grösser sein als gegen sein Ende hin. Damit trägt man dem Umstand Rechnung, dass bei zunehmender Distanz zur Zuführung immer weniger unerwünschte Partikel auftreten.

## Patentansprüche

1. Vorrichtung (1) zur automatischen Bestimmung von Kenngrössen an textilem Prüfgut (13) wie Garnen, Vorgarnen und Bändern, beinhaltend
einen Pfad (8), durch den das Prüfgut (13) in seiner Längsrichtung bewegbar ist, mindestens ein entlang des Pfades (8) angeordnetes Messorgan (4, 5, 6) zur Messung eines Parameters des Prüfguts (13),
eine Vorschubeinrichtung (3) zur Erzeugung einer Längsbewegung des Prüfguts (13) im Pfad,
eine Zuführung (2) zum Zuführen des Prüfguts (13) in den Pfad (8) und
Mittel zur Entfernung von unerwünschten Partikeln wie losen Fasern, Schmutz oder Staub,
**dadurch gekennzeichnet, dass**
die Mittel zur Entfernung von unerwünschten Partikeln mehrere entlang des Pfades (8) angeordnete und voneinander beabstandete Absaugorgane (14-18, 31, 32, 40, 41) beinhalten, die eingerichtet sind, einen Unterdruck zu erzeugen, mittels welchen die unerwünschten Partikel aus dem Pfad (8) absaugbar sind.

2. Vorrichtung (1) nach Anspruch 1, **dadurch gekennzeichnet, dass** zumindest eine Untermenge der Absaugorgane (14-18) derart ausgebildet und angeordnet ist, dass im Pfad (8) eine Luftströmung aufbaubar ist, die das Prüfgut (13) erfasst.

3. Vorrichtung (1) nach Anspruch 2, **dadurch gekennzeichnet, dass** längs des Pfades (8) ein Messkanal (9) mit mindestens einem Messorgan (25) vorgesehen ist und die Untermenge der Absaugorgane (14-18) und der Messkanal (9) so aufeinander abgestimmt sind, dass die Luftströmung in mindestens einem Kanalabschnitt die gesamte Kanalwand (27) überströmt.

4. Vorrichtung nach einem der Ansprüche 1-3, **dadurch gekennzeichnet, dass** die Mittel zur Entfernung von unerwünschten Partikeln einen sich entlang des Pfades (8) erstreckenden Luftkanal (14) beinhalten, in dem mehrere als Absaugorgane ausgebildete Öffnungen (15, 16, 17, 18) angeordnet sind.

5. Vorrichtung nach einem der vorangehenden Ansprüche, **dadurch gekennzeichnet, dass** mindestens ein Absaugorgan (30, 31) als mindestens ein Rohrstück mit einer Öffnung ausgebildet ist, die auf eine Zone im Bereich des Pfades (8) ausgerichtet ist.

6. Vorrichtung nach einem der Ansprüche 1-5, **dadurch gekennzeichnet, dass** ein Absaugorgan (16) im Bereich eines Messorgans (25) angeordnet ist.

7. Vorrichtung nach einem der Ansprüche 1-5, **dadurch gekennzeichnet, dass** ein Absaugorgan (17, 18) ausserhalb des Bereichs eines Messorgans (25) angeordnet ist.

8. Vorrichtung nach einem der vorangehenden Ansprüche, **dadurch gekennzeichnet, dass** ein Absaugorgan (40, 41) im Bereich der Zuführung (2) des Prüfguts (13) angeordnet ist.

9. Vorrichtung nach einem der vorangehenden Ansprüche, **dadurch gekennzeichnet, dass** ein Absaugorgan als Ventilator (42) ausgebildet ist.

10. Verfahren zur Entfernung von unerwünschten Partikeln wie losen Fasern, Schmutz oder Staub aus einer Vorrichtung (1) zur automatischen Bestimmung von Kenngrössen an textilem Prüfgut (13) wie Garnen, Vorgarnen und Bändern, in welcher Vorrichtung (1) das Prüfgut (13) entlang eines Pfades (8) in seiner Längsrichtung bewegt und ein Parameter des Prüfguts (13) gemessen wird,
**dadurch gekennzeichnet, dass**
an mehreren voneinander beabstandeten Stellen entlang des Pfades (8) ein Unterdruck erzeugt wird, mittels welchen die unerwünschten Partikel aus dem Pfad (8) abgesaugt werden.

11. Verfahren nach Anspruch 10, **dadurch gekennzeichnet, dass** im Pfad (8) eine Luftströmung aufgebaut wird, die das Prüfgut (13) erfasst.

12. Verfahren nach Anspruch 11, **dadurch gekennzeichnet, dass** längs des Pfades (8) ein Messkanal (9) mit mindestens einem Messorgan (25) bereitgestellt wird und die Luftströmung in mindestens einem Kanalabschnitt die gesamte Kanalwand (27) überströmt.

## Claims

1. Device (1) for the automatic determination of characteristics on textile test material (13), such as yarns, rovings and slivers, containing
a path (8), through which the test material (13) is movable in its longitudinal direction, at least one measuring element (4, 5, 6) arranged along the path (8) for measuring a parameter of the test material (13),
a feed mechanism (3) for generating a longitudinal movement of the test material (13) in the path,
a supply device (2) for supplying the test material (13) into the path (8), and
means for removing undesired particles, such as loose fibres, dirt or dust,
**characterised in that**
the means for removing undesired particles contain a plurality of suction elements (14 to 18, 31, 32, 40, 41) which are arranged along the path (8) and spaced apart from one another and are configured for generating an underpressure, by means of which the undesired particles are suckable from the path (8).

2. Device (1) according to claim 1, **characterised in that** least one subset of suction elements (14 to 18) is configured and arranged in such a way that an air flow, which engages the test material (13), can be built up in the path (8).

3. Device (1) according to claim 2, **characterised in that** a measuring channel (9) comprising at least one measuring element (25) is provided along the path (8) and the subset of suction elements (14 to 18) and the measuring channel (9) are matched to one another in such a way that the air flow in at least one channel portion flows over the entire channel wall (27).

4. Device according to any one of claims 1 to 3, **characterised in that** the means for removing undesired particles contain an air channel (14) extending along the path (8), in which channel a plurality of openings (15, 16, 17, 18) configured as suction elements are arranged.

5. Device according to any one of the preceding claims, **characterised in that** at least one suction element (30, 31) is configured as at least one tubular piece with an opening, which is aligned with a zone in the region of the path (8).

6. Device according to any one of claims 1 to 5, **characterised in that** a suction element (16) is arranged in the region of a measuring element (25).

7. Device according to any one of claims 1 to 5, **characterised in that** a suction element (17, 18) is arranged outside the region of a measuring element (25).

8. Device according to any one of the preceding claims, **characterised in that** a suction element (40, 41) is arranged in the region of the supply device (2) of the test material (13).

9. Device according to any one of the preceding claims, **characterised in that** a suction element is configured as a ventilator (42).

10. Method for removing undesired particles such as loose fibres, dirt or dust from a device (1) for the automatic determination of characteristics on textile test material (13) such as yarns, rovings and slivers, in which device (1) the test material (13) is moved along a path (8) in its longitudinal direction and a parameter of the test material (13) is measured,
**characterised in that**
an underpressure, by means of which the undesired particles are sucked from the path (8), is generated at a plurality of points, which are spaced apart from one another, along the path (8).

11. Method according to claim 10, **characterised in that** an air flow, which engages the test material (13), is built up in the path (8).

12. Method according to claim 11, **characterised in that** a measuring channel (9) comprising at least one measuring element (25) is provided along the path (8) and the air flow flows over the entire channel wall (27) in at least one channel portion.

## Revendications

1. Dispositif (1) pour déterminer automatiquement des grandeurs caractéristiques d'une matière textile à contrôler (13) telle que des fils, des mèches et des rubans, comprenant un trajet (8) sur lequel la matière à contrôler (13) peut être déplacée dans le sens de sa longueur,
au moins un organe de mesure (4, 5, 6) disposé le long du trajet (8) pour mesurer un paramètre de la matière à contrôler (13),
un dispositif d'avancement (3) pour produire un mouvement longitudinal de la matière à contrôler (13) dans le trajet,
une arrivée (2) pour amener la matière à contrôler (13) dans le trajet (8) et
des moyens pour enlever les particules indésirables telles que fibres libres, salissures ou poussières,
**caractérisé en ce que**
les moyens pour enlever les particules indésirables comprennent plusieurs organes d'aspiration (14-18, 31, 32, 40, 41) disposés le long du trajet (8) et écartés les uns des autres, qui sont conçus pour produire une dépression au moyen de laquelle les particules indésirables peuvent être aspirées hors du trajet (8).

2. Dispositif (1) selon la revendication 1, **caractérisé en ce qu'**au moins un sous-ensemble des organes d'aspiration (14-18) est conçu et disposé de telle façon qu'un écoulement d'air saisissant la matière à contrôler (13) peut être créé dans le trajet (8).

3. Dispositif (1) selon la revendication 2, **caractérisé en ce qu'**il est prévu le long du trajet (8) un conduit de mesure (9) avec au moins un organe de mesure (25) et le sous-ensemble des organes d'aspiration (14-18) et le conduit de mesure (9) sont adaptés l'un à l'autre de telle manière que l'écoulement d'air parcourt toute la paroi du conduit (27) dans au moins une section du conduit.

4. Dispositif selon l'une des revendications 1 à 3, **caractérisé en ce que** les moyens pour enlever les particules indésirables comprennent un conduit d'air (14) s'étendant le long du trajet (8), dans lequel sont disposés plusieurs ouvertures conçues comme des organes d'aspiration (15, 16, 17, 18).

5. Dispositif selon l'une des revendications précédentes, **caractérisé en ce qu'**au moins un organe d'aspiration (30, 31) est conçu comme au moins un morceau de tuyau avec une ouverture qui est orientée vers une zone dans la région du trajet (8).

6. Dispositif selon l'une des revendications 1 à 5, **caractérisé en ce qu'**un organe d'aspiration (16) est disposé dans la région d'un organe de mesure (25).

7. Dispositif selon l'une des revendications 1 à 5, **caractérisé en ce qu'**un organe d'aspiration (17, 18) est disposé en dehors de la région de l'organe de mesure (25).

8. Dispositif selon l'une des revendications précédentes, **caractérisé en ce qu'**un organe d'aspiration (40, 41) est disposé dans la région de l'arrivée (2) de la matière à contrôler (13).

9. Dispositif selon l'une des revendications précédentes, **caractérisé en ce qu'**un organe d'aspiration est conformé comme un ventilateur (42).

10. Procédé pour enlever des particules indésirables telles que fibres libres, salissures ou poussières d'un dispositif (1) pour déterminer automatiquement des grandeurs caractéristiques d'une matière textile à contrôler (13) telle que des fils, des mèches et des rubans, dispositif (1) dans lequel la matière à contrôler (13) est déplacée dans le sens de sa longueur le long d'un trajet (8) et un paramètre de la matière à contrôler (13) est mesuré,
**caractérisé en ce**
**qu'**une dépression est produite en plusieurs endroits distants les uns des autres le long du trajet (8), au moyen de laquelle les particules indésirables sont aspirées hors du trajet (8).

11. Procédé selon la revendication 10, **caractérisé en ce qu'**il se crée dans le trajet (8) une circulation d'air qui saisit la matière à contrôler (13).

12. Procédé selon la revendication 11, **caractérisé en ce qu'**il est prévu le long du trajet (8) un conduit de mesure (9) avec au moins un organe de mesure (25) et l'écoulement d'air parcourt toute la paroi du conduit (27) dans au moins une section du conduit.
